# EUROPÄISCHE PATENTSCHRIFT

(11) **EP 0 696 456 B1**
(45) Veröffentlichungstag und Bekanntmachung des Hinweises auf die Patenterteilung: **14.03.2001**
(21) Anmeldenummer: 95107299.0
(22) Anmeldetag: 13.05.1995
(51) Int. Cl.: A61K 39/395

(54) **Kombination von Nekrose-induzierenden Substanzen mit Substanzen, die durch Nekrosen aktiviert werden, zur selektiven Therapie von Tumoren und entzündlichen Erkrankungen**
Combination of necrosis inducing substances with substances which are activated by necrosis for the selective treatment of tumours and or inflammatory diseases
Combinaison de substances induisant de la nécrose avec des substances qui sont activées par la nécrose pour le traitement sélectif de tumeurs et de maladies inflammatoires

(30) Priorität: 20.05.1994 DE 4417865
(43) Veröffentlichungstag der Anmeldung: 14.02.1996
(73) Patentinhaber: HOECHST AKTIENGESELLSCHAFT, 65929 Frankfurt am Main (DE)
(72) Erfinder: Bosslet, Klaus, Dr., D-35037 Marburg (DE); Czech, Jörg, Dr., D-35041 Marburg (DE); Hoffmann, Dieter, Dr., D-35041 Marburg (DE)
(74) Vertreter: Fischer, Hans-Jürgen, Dr.

(56) Entgegenhaltungen:
- EP-A- 0 540 859
- EP-A- 0 595 133
- WO-A-92/17151
- US-A- 5 098 702
- CHEMICAL ABSTRACTS, vol. 119, no. 19, 8.November 1993 Columbus, Ohio, US; abstract no. 195291b, MELTON R.G. ET AL.: "Tumor necrosis factor increases tumor uptake of co-administered antibody-carboxypeptidase G2 conjugate." Seite 43; XP002076132 & MELTON R.G. ET AL.: "Tumor necrosis factor increases tumor uptake of co-administered antibody-carboxypeptidase G2 conjugate." EUR. J. CANCER, PART A, Bd. 29A, Nr. 8, 1993, Seiten 1177-1183,

## Beschreibung

Die Erfindung betrifft eine Kombination von bei Tumoren oder entzündetem Gewebe, d.h. prolifierendem Endothel, nekrose-induzierenden Substanzen (Komponente I) mit anderen untoxischen Substanzen ("Prodrugs", Komponente II). Die durch nekrotische Prozesse freiwerdenden Enzyme spalten dann die untoxische "Prodrug" in die toxische "Drug", was zu massivem Tumorzelltod bzw. Entzündungsrückgang führt.

Zur Behandlung fortgeschrittener (metastasierter) solider Tumore steht heutzutage nur die Chemotherapie zur Verfügung. Diese Therapieform bewirkt zwar bei einer Reihe solider Tumoren einen objektivierbaren antitumoralen Effekt jedoch nur bei meist schweren Nebenwirkungen für den Patienten. Aus dieser unbefriedigenden Situation heraus ergibt sich die dringende Notwendigkeit, bessere und nebenwirkungsarme Therapieformen zu entwickeln.

In den letzten Jahren wurden mehrere Versuche unternommen, um basierend auf spezifischen immunologischen Erkennungsmechanismen neue und effiziente Therapien in die Klinik einzuführen. Hierbei handelt es sich vor allem um Therapieansätze, bei denen mittels Antikörpern, die für Oberflächenstrukturen auf Tumoren selektiv sind, toxische Prinzipien zum Tumorgewebe transportiert werden sollten. Bei diesen klinischen Versuchen stellte sich heraus, daß Makromoleküle, wie z.B. monoklonale Antikörper oder deren rekombinante Varianten, zwar sehr selektiv an die Tumorzellen in vivo binden, aber nur in einem sehr geringen, für die Therapie meist nicht ausreichendem Maße. Diese wissenschaftlichen Erkenntnisse führten zur Entwicklung sogenannter Mehrstufenkonzepte, bei denen an einen tumorselektiven monoklonalen Antikörper ein Enzym gekoppelt wurde (Antikörperenzymkonjugat), welches in der Lage ist, nach einer entsprechenden Prälokalisationsphase am Tumor (1. Schritt) eine danach injizierte untoxische niedermolekulare Prodrug (2. Schritt) tumorselektiv zu einer toxischen Drug zu spalten. Präklinisch führt dieses Zweiphasenkonzept zu höheren Drugkonzentrationen am Tumor als die konventionelle Chemotherapie, was mit verbesserter Wirksamkeit im Humantumor-Xenograftmodell in der nackten Maus einhergeht (Senter, P.D. et al., Bioconj. Chem. 4: 3-9, 1993). Neben der zu erwartenden Immunogenität der verwendeten Antikörperenzymkonjugate (Maus-Antikörper verbunden mit xenogenen, meist bakteriellen Enzymen) sind ihre Penetrationsprobleme durch den soliden Tumor sowie ihre Restriktion auf bestimmte Tumortypen, abhängig von der Präsenz des erkannten tumorassoziierten Antigens zu nennen. Hieraus resultiert die Notwendigkeit, verschiedene Antikörperenzymkonjugate mit unterschiedlicher Spezifität zu entwickeln, um wenigstens die häufigsten Tumore (Gastrointestinaltrakt-, Lungen-, Mamma-, Ovar-, Prostatakarzinome) therapieren zu können.

Im folgenden wird nun eine pharmazeutische Zusammensetzung vorgestellt, welches überraschenderweise die oben aufgezeigten Probleme der Immunogenität, Heterogenität und mangelnden Tumorpenetration nicht besitzt, aber die Vorteile des Zweiphasenkonzeptes, die verbesserte Drugkonzentrierung am Tumor, besitzt und demzufolge eine universell einsetzbare nebenwirkungsarme Therapie solider Tumore ermöglicht.

Die pharmazeutische Zusammensetzung besteht aus zwei Komponenten, einer Komponente, die tumorselektiv Nekrosen induziert oder selektiv toxisch ist für proliferierendes Endothel (Tumorendothel oder Endothel in entzündlichen Prozessen) und einer untoxischen Prodrugkomponente, die durch während nekrotischer Prozesse freiwerdende Enzyme zu einer toxischen Drug gespalten wird. Die Nekrose-induzierende I. Komponente kann ein Inhibitor des Tumor- oder Endothelzellstoffwechsels sein oder eine Tumorendothel-spezifische Substanz. Die Tumorendothel-spezifische oder die Nekrose-induzierende I. Komponente wird parenteral, vorzugsweise intravenös appliziert. Sie bindet im Falle der Tumorendothel-spezifischen Komponente selektiv an ein Epitop, welches präferentiell auf proliferierendem Endothel der Blutgefäße exprimiert ist, ohne das Tumorinterstitium durchdringen zu müssen.

Nach Internalisation der Komponente in die proliferierenden Endothelzellen oder durch Aktivierung zytotoxischer Wirtseffektormechanismen bei Nichtinternalisation wird das proliferierende Endothel in seinem Wachstum inhibiert bzw. gänzlich zerstört. Hieraus resultiert eine Nährstoffunterversorgung zahlreicher Tumorzellen, was zur Wachstumsinhibition und zum Absterben bestimmter Tumorareale führt. Vergleichbare Effekte können auch durch i.v. injizierbare Tumorstoffwechsel- oder Endothelstoffwechsel-hemmende Substanzen erzielt werden. Bei der so entstehenden Nekrose werden intrazelluläre Enzyme, vorzugsweise lysosomale Glykosidasen frei, welche die dann vorzugsweise i.v. zu injizierende II. Komponente, die untoxische Prodrug, zur toxischen Drug spalten. Die so im Tumor erzeugten hohen Drugkonzentrationen führen dann zu massivem Tumorzelltod, dem überlegenen antitumoralen Effekt dieser neuen Zweikomponententherapie bzw. Entzündungsrückgang.

Als I. Komponente des neuen Zweikomponentensystems können verschiedene im folgenden näher beschriebene Substanzen verwendet werden:
**a) Monoklonale Antikörper (MAk), selektiv für proliferierendes Endothel bzw. deren humanisierte Varianten mit Komplement-aktivierenden und/oder die "Antibody-Dependent Cell-mediated Cytotoxicity" ADCC vermittelndem Fc-Teil (Wirkungsweise über Zellzerstörung) sowie nicht zytolytische Varianten oder Fragmente (Auslösung von Apoptose);**
**b) Immunkonjugate aus wie unter a) beschriebenen Substanzen, verbunden mit Toxinen oder toxischen Chemikalien;**
**c) Ligandtoxine spezifisch für proliferierendes Endothel, bestehend aus einem Rezeptorliganden verbunden mit Toxinen bzw. mit toxischen Chemikalien;**
**d) Tumorzell- oder Endothelzellstoffwechsel-inhibierende Substanzen, die zumindest Teile von Tumorzellpopulationen jeweils lokal zerstören.**

Die unter **a)** beschriebenen monoklonalen Antikörper sind vorzugsweise spezifisch für ein proliferationsabhängiges Endothelantigen, wie z. B. der VEGF-Rezeptor (Terman et al., 1991, Oncogene 6, 1677-1683; Ullrich und Schlesinger, 1990, Cell 61, 203-212; Millauer et al., 1993, Cell 72, 835-846; Millauer et al., 1994, Nature 367 (6463) 576-579; Kaipainen et al., 1993, J. Exp. med. 178 (6), 2077-2088; Plate et al., 1993, Cancer Res. 53, 5822-5827), die von Clarke und West (Electrophoresis, 12 (7-8), 500-508, 1991) beschriebenen Antigene auf Endothel, das von Hagemeier et al. (Int. J. Cancer 38 (4) 481-488, 1986) beschriebene 30.5 kDa Tumorendothelspezifische Antigen, ein auf proliferierenden Endothelzellen vorkommendes Apoptose-mediierendes Antigen wie beispielsweise der Vitronektinrezeptor (Integrin αᵥβ₃) (Brooks et al., Science, 264, 569-71, 1994; Brooks et al., Cell 79, 1157-64, 1994), der VEGF/VEGF-Rezeptor-Komplex (VEGF/FLK1, VEGF/KDR) (Abraham et al., US 52 19739, Abraham et al., WO 91 02058A, Millauer et al., Cell 72, 835-846, 1993; Terman et al., Oncogene 6, 1677-1683, 1991, Terman et al., Biochem. Biophys. Res. Comm., 187, 1579-1586, 1992), die Fibronektin-CH₂-Domaine (Carnemolla et al., J. Cell Biol. 108, 1139-1148, 1989, Castellani et al., Int. J. Cancer, 59, 612-618, 1994), das Endoglin (Fernandez-Ruiz et al., Cytogenet. Cell Genet. 64, 204-207, 1993; Gougos et al., J. Biol. Chem. 265, 8361-8364, 1990), das Endosialin (Garin-Chesa und Rettich, USP 5 342 757), die in WO 94/10331 mittels MAk definierten, auf proliferierenden Endothelien vorkommenden Antigene, das EAM-1 Sialoglycoproteinantigen (EPA 0583 799A), das FLK-2 Protein (WO 94/01576), das CMP-170 Antigen (EPA 0 585 963 A1), das E9 Antigen (Wang et al., Int. J. Cancer 54, 363-370, 1993, Wang et al., J. Immunol. Methods 171, 55-64, 1994), das neue 180 kDa dermale Endothelzell-Aktivierungsantigen (Westphal et al., J. Invest. Dermatol. 100, 27-34, 1993), das FLt Protein (Shibuya et al., Oncogene 5, 519-524, 1990; DeVries et al., Science 255, 989-991, 1992), der PDGF-Rezeptor β (Plate et al., Laboratory Investigation, 67, 529-534, 1992), der PDGF/PDGF-Rezeptor β-Komplex, der PDEGF/PDEGF-Rezeptorkomplex (Ishikawa et al., Nature 338, 557-562, 1989), das induzierbare, Bluthirnschrankenendothel-spezifische Antigen HT7 (Neurothelin, Basignin; gp 42, OX 47) (Seulberger et al., Annals of the New York Academy of Sciences, 633, 611-614, 1991; Seulberger et al., Neuroscience Letter 140, 93-97, 1992). Zusätzlich zu den oben definierten MAk können auch die von Burrows und Thorpe (Pharmac. Ther. 64, 155-174, 1994) erwähnten MAk verwendet werden. Alle in **a)** erwähnten MAk können auch zur Herstellung von Fusionsproteinen in Verbindung mit procoagulierenden Faktoren (Denekamp, Cancer Topics 6, 6-8, 1986) oder Zytokinen bzw. Chemokinen (Mulligan, Science 260, 926-932, 1993) verwendet werden. Verbunden mit Vektor-DNA, welche für proinflammatorische, immunregulatorische oder proliferationsinhibierende Proteine codiert, können vor allem die unter **a)** beschriebenen MAk, welche internalisieren, zum "Targeting" und zur Modifikation des proliferierenden Endothels benutzt werden (Nabel et al., Science 249, 1285-1288, 1990). Im Prinzip kann eine Internalisierung, wenn nicht durch das erkannte Antigen selbst vermittelt, durch die Gabe eines zweiten Antikörpers mit Spezifität gegen den ersten bewirkt werden. Anstelle von klassischen, aus Hybridomen gewonnenen MAk können selbstverständlich auch aus nicht-humanen oder humanen Genbanken über "display libraries" gewonnene Antikörper (Little et al., Biotech Adv. 12, 539-555, 1994) sowie deren Derivate wie z.B. "Single Chain Fragment Variable Region" (scFvs), "Domain Antibodies" (dAbs), Antigen-bindende Peptide oder Peptidmimetika obiger Spezifitäten verwendet werden. Antigenbindende Peptide sind insbesondere die bei Brooks et al. (S. 3 loc cit.) aufgeführten zyklischen Peptide, die Apoptose über Bindung an den Vitronektinrezeptor auslösen.

Die unter **b)** beschriebenen Immunkonjugate werden dadurch erzeugt, daß die unter **a)** definierten spezifischen Antikörper oder deren Varianten mit Toxinen xenogener Herkunft (Ricin A, Diphtherie-Toxin A, Pseudomonas exotoxin A; Burrows and Thorpe, PNAS 90, 8996-9000, 1993) oder humaner Herkunft (Angiogenin, RNAsen; Rybak et al., PNAS 89, 3165-3169, 1992) verknüpft sind. Weiterhin kann auch eine Verknüpfung mit toxischen Synthetika oder Naturstoffen, wie z. B. Alkylantien, Antimetaboliten, Anthrazyklinen, Vincaalkaloiden, Taxol, Chalichimycin etc., sowie Radioisotopen, vorzugsweise α- oder β-Strahlern in komplexierter Form (z.B.: Y-90 DOTA:2-(p-Nitrobenzyl)-1,4,7,10-tetracyclododecan-N,N',N'',N''',N''''-tetraessigsäure; Moi et al., J. Am. Chem. Society, **110**, 6266 (1988)) durchgeführt werden.

Bei den unter **c)** beschriebenen Ligandtoxinen handelt es sich um natürliche oder synthetische Liganden, welche an die in **a)** mittels MAk definierten Rezeptoren oder Antigene binden, vorzugsweise um Varianten des "Vascular Endothelial Growth Factor" (VEGF) (Shweiki et al. 1993, J. Clin. Invest. 91, 2235-2243), verbunden mit den unte**r b)** beschriebenen Toxinen und toxischen Substanzen sowie um VEGF-Antagonisten oder Agonisten.

Im Falle der Tumorzell- oder Endothelzellstoffwechsel-inhibierenden Substanzen (**d**) können z.B. 5,6-Dimethyl-Xanthenon Essigsäure oder Flavonessigsäure (Zwi et al., Pathology 26, 161-169, 1994), Zilascorb (5,6-O-benzyliden-d-L-ascorbinsäure; Pettersen et al., Brit. J. Cancer 67, 650-656, 1993) oder AGM 1470 (Antoine et al., Cancer Res. 54, 2073-2076, 1994) benutzt werden. Weitere Beispiele für Tumorzellstoffwechsel-inhibierende Substanzen sind Immunkonjugate oder Fusionsproteine, wobei diese Konstrukte selektiv für ein internalisierbares tumorassoziiertes Antigen sind, verbunden mit einer toxischen Komponente, vorzugsweise einem Stoffwechsel-hemmenden Enzym, z.B. Pseudomonas Exotoxin (Brinkmann et al., Proc. Natl. Acad. Sci. USA, 88, 8616-8620, 1991), einem toxischen Synthetikum wie Cyclophosphamid oder toxischem Naturstoff, z.B. Daunomycin.

Die Injektion der unter a) - d) beschriebenen jeweiligen I. Komponenten in einen Patienten bewirkt also eine selektive Gewebenekrose entweder durch direkte Schädigung des Tumorgewebes (d) oder indirekt durch Beeinflussung des proliferierenden Endothels (a, b, c).

Zur vollständigen Berücksichtigung des Standes der Technik sind die im folgenden Abschnitt zusammengefaßten Schriften zu berücksichtigen.

Gemäß EP 0 540 859 werden Prodrugs von Anthrazyclin Typ durch tumorspezifische Antikörper-Enzymkomplexe in cytotoxisch wirksame Verbindungen umgewandelt. In WO 92/17151 wird eine Methode beansprucht, um pharmakologisch aktive Substanzen parazellulär durch epotheliales Gewebe von Säugetieren zu verabreichen. Dazu wird durch ausgewählte Substanzen, die mit dem Tumor-Nekrose-Faktor wechselwirken, in Epithelzellschichten, welche den Tumor-Nekrose-faktor exprimieren, die Resistenz der "tight junctions" herabgesetzt. Dieser Effekt wird dann ausgenutzt, um pharmakologisch aktive Substanzen durch die Epithelschicht zu schleusen. Im US Patent 5 098 702 wird eine Mischung aus TNF und IL-2 in synergistisch wirksamen Mengen zur Behandlung von Krebs in Säugetieren beansprucht. In Chemical abstracts, vol. 119, No. 19, vom 8. Nov. 1993 weist Meltron, et al. darauf hin, daß TNF die Aufnahme von gleichzeitig verabreichtem Antikörper-Carboxypeptidase G2 Konjugat in Tumore erhöht. Dieselben Autoren (Melbron et al.) publizieren denselben Befund auch in Eur. J. Cancer, Part A, Bd. 29A, Nr. 8, 1993, Seiten 1177-1183. In EP 0 595 133 A werden Prodrugs mit Glykosyl-Spacern sowie deren Herstellung und Verwendung beschrieben.

Gleichzeitig und/oder nach Auftreten der Nekrose werden als II. Komponente Prodrugs injiziert, die durch Enzyme, die während der Tumorzellnekrose freigesetzt werden, spaltbar sind, beispielsweise die in EP-A 0 511 917 A1 bzw. EP-A 0 595 133 beschriebenen Prodrugs, vorzugsweise die bei Bosslet et al. (Cancer Res. 54, 2151-2159, 1994) in Fig. 3 beschriebene N-(4-β-glucuronyl-3-nitrobenzyloxycarbonyl)-doxorubicin Prodrug (in den Beispielen "Prodrug" genannt).

Im folgenden werden tierexperimentelle Beispiele beschrieben, welche die überlegene pharmakodynamische Wirksamkeit der neuen Zweikomponententherapie belegen. Diese Tiermodelle besitzen eine hohe Prädiktivität für die klinische Situation.

### Beispiel 1:

Das von Burrows und Thorpe (Proc. Natl. Acad. Sci., USA, 90, 8996-9000, 1993) aufgebaute Mausmodell wurde verwendet, um die überlegene pharmakodynamische Wirksamkeit eines anti Tumorendothel-lmmuntoxins (I. Komponente) zusammen mit der Prodrug (F 826) als II. Komponente zu beweisen.

### Das Maus-Modell ist wie folgt aufgebaut:

Eine Mischung von 1.4 x 10⁷ C1300 Maus-Neuroblastomzellen und 6 x 10⁶ C1300 Muγ Zellen (mit Maus γ-Interferon transfizierte Maus-Neuro-blastomlinie) wurden subkutan in die rechte Flanke von Balb/c nu/nu-Mäusen injiziert. Vierzehn Tage später, als die Tumore einen Durchmesser von 0.8 - 1.2 cm erreicht hatten, wurden die Tiere in vier Gruppen zu je 6 Tieren randomisiert. Die Tiere erhielten am Tag 15 das Immuntoxin und an den Tagen 17, 20 und 23 die Prodrug i.v. injiziert.

Das injizierte Tumorendothel-Immuntoxin besteht aus einem monoklonalen Antikörper (MAk M 5/114), der spezifisch für Maus MHC-Klasse II Moleküle des Haplotyps d ist, verbunden mit der deglykosilierten Ricin-A-Kette. Normale Endothelzellen von Balb/c nu/nu-Mäusen exprimieren keine MHC-Klasse II Moleküle. Das von den C1300 Muγ Tumorzellen in vivo lokal freigesetzte γ-Interferon führt allerdings im Tumor zu einer Aktivierung der dort befindlichen Endothelzellen, verbunden mit einer deutlichen Expression von MHC-Klasse II Molekülen. Hierdurch wird ein quasi Tumorendothel-spezifisches Antigen erzeugt (abgesehen von Maus B-Zellen, Makrophagen und einigen epithelialen Zellen, die MHC-Klasse II Antigene konstitutiv exprimieren). Die Injektion des Immuntoxins sollte demzufolge zu einer präferentiellen Bindung an die Tumorendothel-Zellen führen, gefolgt von Endothelzerstörung nach Internalisation des Konjugates. Die aus der Tumorendothelzerstörung resultierende Nekrose führt zu einer Freisetzung intrazellulärer Enzyme, vor allem aber der β-glucuronidase, welche die nachfolgend verabreichte Prodrug im Tumor aktivieren sollte.

Um diese Hypothese zu belegen wurden die in vier Gruppen verteilten Experimentaltiere, wie in Tabelle 1 beschrieben, behandelt.

**Tabelle 1**

| | Behandlungsschema | |
|---|---|---|
| Gruppe | Immuntoxin; µg/mouse | Prodrug; mg/mouse |
| 1 | 4 | 4 |
| 2 | 4 | 0 |
| 3 | 0 | 4 |
| 4 | 0 | 0 |

Das Tumorwachstum wurde bestimmt, indem zwei senkrecht aufeinanderstehende Durchmesser des Tumors mittels einer Schieblehre an jedem Tag des Experimentes gemessen wurden. Das Tumorvolumen wurde mit Hilfe der Formel V = ½ ab² bestimmt, wobei a der längste und b der kürzeste Durchmesser bedeutet.

Die Tumore der Experimentalgruppe 4 zeigten progressives Wachstum. Durch die dreimalige Behandlung mit Prodrug (Gruppe 3) bzw. mit Immuntoxin (Gruppe 2) wurden starke Tumorwachstum-inhibitorische Effekte erzielt. Alle Tiere der Gruppe 1 wurden in eine komplette Regression therapiert. Dieses Experiment belegt die überlegene Wirksamkeit der neuen Zweikomponententherapie mit Immuntoxin und Prodrug. Anstatt des hier verwendeten Immuntoxins mit Spezifität für HLA-Antigene der Maus (Burrows und Thorpe, PNAS 90, 8996-9000, 1993) können im Patienten die unter **a)**, **b)** und **c)** beschriebenen Komponenten eingesetzt werden.

### Beispiel 2:

In 2 weiteren unabhängigen Experimenten wurde die pharmakodynamische Überlegenheit der neuen Zweikomponenten-Therapie belegt. Experimentalgruppen mit jeweils 6 Nacktmäusen wurden mit dem LoVo-Kolonkarzinom bzw. mit dem Mx-1 Mammakarzinom transplantiert. Nachdem die Tumore einen ⌀ von ≈ 5 mm erreicht hatten, wurde am Tag 1-7 Zilascorb (Pettersen et al. 1993, Brit. J. Cancer, 67, 650-656; Borretzen et al., USP 4874 780, 1989) und an den Tagen 8,11 und 14 die Prodrug F 826 i.v. appliziert. Das Behandlungsschema ist in Tabelle 2 näher beschrieben.

**Tabelle 2**

| | Behandlungsschema | |
|---|---|---|
| MX-1 | Zilascorb; mg/Maus | Prodrug; mg/Maus |
| Gruppe 1 | 0.4 | 4 |
| 2 | 0.4 | 0 |
| 3 | 0 | 4 |
| 4 | 0 | 0 |

| LoVo | | |
|---|---|---|
| Gruppe 1 | 0.8 | 4 |
| 2 | 0.8 | 0 |
| 3 | 0 | 4 |
| 4 | 0 | 0 |

Das Tumorwachstum wurde, wie in Beispiel 1 beschrieben, bestimmt und über 30 Tage nach der Therapie verfolgt. Folgende tumortherapeutische Effekte wurden beobachtet:

Die Experimentalgruppen 4, welche mit physiologischer Kochsalzlösung behandelt wurden, zeigten über den Behandlungszeitraum von 38 Tagen progressives Wachstum. Bei einem Tumordurchmesser von ≥ 20 mm wurden die Tiere aus ethischen Gründen getötet. In den Experimentalgruppen 2 und 3 war eine signifikante Hemmung des Tumorwachstums zu beobachten. In mehr als 50 % der Tiere der Behandlungsgruppen 1 wurden Tumorregressionen erreicht.

Diese Beobachtungen zeigen, daß die neue Zweikomponententherapie mit Zilascorb und Prodrug F 826, im Vergleich zur Monotherapie mit einer der Komponenten, überlegene tumortherapeutische Wirksamkeit hat. In weiteren präklinischen in vivo Modellen, in denen humane Lungen-, Brust-, Prostata-, Pankreas- und Magenkarzinome verwendet wurden, konnten vergleichbare Effekte wie für den MX-1 und den LoVo-Tumor beobachtet werden. Unter Verwendung von Flavonessigsäure bzw. 5,6-Dimethyl-Xanthenon Essigsäure als Erstkomponente wurden vergleichbare Ergebnisse wie mit Zilascorb erhalten. Diese präklinischen in vivo Experimente mit hoher Prädiktivität für die klinische Situation zeigen, daß die neue Zweikomponenientherapie mit Tumorzell- bzw. Endothelzellstoffwechsel-inhibierenden Substanzen kombiniert mit Prodrug bei einer breiten Palette menschlicher Tumore unterschiedlichen Ursprungs verwendet werden kann. Im Patienten würden analog zum Tierexperiment die unter **d)** beschriebenen Reagenzien als I. Komponente injiziert werden, gefolgt von F 826 als II. Komponente.

### Beispiel 3:

In einem weiteren unabhängigen Experiment wurde die pharmakodynamische Überlegenheit der neuen Zweikomponententherapie belegt. Experimentalgruppen mit je 6 Tieren wurden mit dem LoVo-Kolonkarzinom transplantiert. Nach die Tumore einen ⌀ von ≈ 5 mm erreicht hatten, wurde am Tag 1 das ScFv PE40 Konstrukt (Brinkmann, Proc. Natl. Acad. Sci., USA, 88, 8616-8620, 1991) in jeweils 12stündigen Abständen insgesamt 4x i.v. appliziert. Die Prodrug wurde an den Tagen 3, 6 und 9 i.v. appliziert. Das Behandlungsschema ist in Tabelle 3 zusammengefaßt.

**Tabelle 3**

| | Behandlungsschema | |
|---|---|---|
| | ScFv PE40 µg/Maus | Prodrug; mg/Maus |
| Gruppe 1 | 5 | 4 |
| 2 | 5 | 0 |
| 3 | 0 | 4 |
| 4 | 0 | 0 |

Folgendes experimentelle Ergebnis wurde erzielt: Im Vergleich zur Kontrollgruppe 4 wurde in der Prodrug-Gruppe 3 eine partielle Tumorregression, im Falle der ScFv PE40-Gruppe 2 lediglich eine Verlangsamung des Tumorwachstums beobachtet. In der Kombinationstherapie Gruppe 1 zeigten 4 von 6 Tieren eine komplette und 2 eine partielle Regression.

Dieses Experiment belegt die überlegene Wirksamkeit der Zweikomponententherapie am Beispiel eines Immuntoxins, welches gegen ein tumorassoziiertes Antigen (TAA) gerichtet ist, in Verbindung mit Prodrug. Für die Anwendung am Patienten bedeutet dieses Experiment, daß eine Zweikomponententherapie mit den unter **d)** beschriebenen Substanzen, verbunden mit der Prodrug F 826, überlegene tumortherapeutische Wirksamkeit ergeben sollte.

Zusammenfassend darf hier festgestellt werden, daß die Kombinationstherapie mit den unter **a)**, **b)**, **c)** und **d)** beschriebenen I. Komponenten und der II. Komponente (Prodrug) überlegene tumortherapeutische Effekte im Vergleich zur Monotherapie mit den Komponenten I oder II erbringt.

## Patentansprüche

1. Pharmazeutische Zusammensetzung, enthaltend ein oder mehrere Wirkstoffe als Komponente I, die Nekrosen in Tumoren und bei entzündlichen Erkrankungen erzeugt und enthaltend ein oder mehrere Prodrugs, die durch Enzyme, die während der Tumorzellnekrose oder bei entzündlichen Erkrankungen, hervorgerufen durch Komponente I, spaltbar sind, als Komponente II, als Kombinationspräparat zur gleichzeitigen, getrennten oder zeitlich abgestuften Anwendung in der zytostatischen oder immunregulierenden Therapie, wobei bei gleichzeitiger Anwendung auch Mischungen der Komponenten I und II umfaßt sind.

2. Pharmazeutische Zusammensetzung nach Anspruch 1, dadurch gekennzeichnet, daß die Komponente I mindestens eine der unter a) - d) beschriebenen Verbindungen enthält.
a) Monoklonale Antikörper (MAk), selektiv für proliferierendes Endothel bzw. deren humanisierte Varianten mit Komplement-aktivierenden und/oder die "Antibody-Dependent Cell-mediated Cytotoxicity" (ADCC) vermittelndem Fc-Teil sowie nicht zytolytische Varianten oder Fragmente;
b) Immunkonjugate aus wie unter a) beschriebenen Substanzen, verbunden mit Toxinen oder toxischen Chemikalien;
c) Ligandtoxine spezifisch für proliferierendes Endothel, bestehend aus einem Rezeptorliganden verbunden mit Toxinen bzw. mit toxischen Chemikalien;
d) Tumorzell- oder Endothelzellstoffwechsel-inhibierende Substanzen, die zumindest Teile von Tumorzellpopulationen jeweils lokal zerstören.

3. Pharmazeutische Zusammensetzung nach Anspruch 1, dadurch gekennzeichnet, daß die Komponente I mindestens einen monoklonalen Antikörper oder einen Rezeptorliganden enthält, der selektiv für proliferierendes Endothel ist, vorzugsweise spezifisch für den VEGFNEGF-Rezeptorkomplex.

4. Pharmazeutische Zusammensetzung nach Anspruch 1, dadurch gekennzeichnet, daß die Komponente I mindestens einen monoklonalen Antikörper oder Liganden oder Antagonisten gegen ein auf proliferierenden Endothelzellen vorkommendes Apoptose-mediierendes Antigen enthält, vorzugsweise das αᵥβ₃ Integrin.

5. Pharmazeutische Zusammensetzung nach Anspruch 1, dadurch gekennzeichnet, daß die Komponente I mindestens ein zytotoxisches ImmunKonjugat enthält.

6. Pharmazeutische Zusammensetzung nach Anspruch 1, dadurch gekennzeichnet, daß die Komponente I mindestens ein Ligandtoxin enthält.

7. Pharmazeutische Zusammensetzung nach Anspruch 1, dadurch gekennzeichnet, daß sie eine Tumorzell- oder Endothelzellstoffwechsel-inhibierende Substanz enthält.

8. Pharmazeutische Zusammensetzung nach Anspruch 1, dadurch gekennzeichnet, daß als II. Komponente die Prodrug N-(4-β-glucuronyl-3-nitrobenzyloxycarbonyl)-doxorubicin verwendet wird.

9. Verwendung von ein oder mehreren Wirkstoffen, die Nekrosen in Tumoren und bei entzündlichen Erkrankungen erzeugen (Komponente I) mit einem oder mehreren Wirkstoffen, die durch die erzeugten Nekrosen aktiviert werden (Komponente II), zur Herstellung eines Medikaments zur Bekämpfung von Tumoren oder entzündlichen Erkrankungen.

## Claims

1. A pharmaceutical composition containing one or more active compounds as component I, which produces necroses in tumors and in inflammatory disorders, and containing, as component II, one or more prodrugs which can be cleared by enzymes set free during tumor cell necrosis or inflammatory disorders induced by component I, as a combination preparation for simultaneous, separate or sequential use in cytostatic or immunoregulating therapy, mixtures of the components I and II also being included in the case of simultaneous use.

2. The pharmaceutical composition as claimed in claim 1, wherein the component I contains at least one of the compounds described under a) - d).
a) monoclonal antibodies (Mab), selectively for proliferating endothelium or its humanized variants having complement-activating and/or the "antibody-dependent cell-mediated cytotixicity" (ADCC) mediating Fc part and also noncytolytic variants or fragments;
b) immunoconjugates of substances described under a) bound to toxins or toxic chemicals;
c) ligand toxins specific for proliferating endothelium, consisting of a receptor ligand bound to toxins or to toxic chemicals;
d) tumor cell- or endothelial cell metabolism-inhibiting substances, which in each case locally destroy at least parts of tumor cell populations.

3. The pharmaceutical composition as claimed in claim 1, wherein the component I contains at least one monoclonal antibody or one receptor ligand which is selective for proliferating endothelium, preferably specifically for the VEGF/VEGF receptor complex.

4. The pharmaceutical composition as claimed in claim 1, wherein the component I contains at least one monoclonal antibody or ligands or antagonists against an apoptosis-mediating antigen occurring on proliferating endothelial cells, preferably the αᵥβ₃ integrin.

5. The pharmaceutical composition as claimed in claim 1, wherein the component I contains at least one cytotoxic immunoconjugate.

6. The pharmaceutical composition as claimed in claim 1, wherein the component I contains at least one ligand toxin.

7. The pharmaceutical composition as claimed in claim 1, which contains a tumor cell- or endothelial cell metabolism-inhibiting substance.

8. The pharmaceutical composition as claimed in claim 1, wherein the prodrug N-(4-β-glucuronyl-3-nitrobenzyloxycarbonyl)doxorubicin is used as IInd component.

9. The use of one or more active compounds which produce necroses in tumors and in inflammatory disorders (component I) with one or more active compounds which are activated by the necroses produced (component II) for the production of a medicament for the control of tumors or inflammatory disorders.

## Revendications

1. Composition pharmaceutique, contenant une ou plusieurs substances actives en tant que composant I, qui produit des nécroses dans des tumeurs et dans des maladies inflammatoires, et contenant, en tant que composant II, un ou plusieurs précurseurs de médicaments qui peuvent être coupés par des enzymes engendrées par le premier composant pendant la nécrose de cellules tumorales ou dans des maladies inflammatoires, en tant que composition à plusieurs composants, pour l'administration simultanée, séparée ou échelonnée dans le temps, dans la thérapie cytostatique ou immunorégulatrice, des mélanges des composants I et II étant également englobés dans le cas d'administration simultanée.

2. Composition pharmaceutique selon la revendication 1, caractérisée en ce que le composant I contient au moins un des composés décrits en a) - d)
a) des anticorps monoclonaux (AcM) sélectifs pour l'endothélium proliférant, ou leurs variants humanisés avec fragment Fc activant le complément et/ou provoquant la "cytotoxicité cellulaire dépendant des anticorps" ADCC *(Antibody-Dependent Cell-mediated Cytotoxicity)* ainsi que leurs fragments ou variants non cytolytiques ;
b) des immunoconjugués à base de substances décrites en a), liées à des toxines ou à des substances chimiques toxiques ;
c) des toxines-ligands spécifiques d'endothélium proliférant, constitués d'un ligand récepteur lié à des toxines ou à des substances chimiques toxiques ;
d) des substances inhibant le métabolismes de cellules tumorales ou de cellules endothéliales, qui détruisent localement chacune au moins des parties de populations de cellules tumorales.

3. Composition pharmaceutique selon la revendication 1, caractérisée en ce que le composant I contient au moins un anticorps monoclonal ou un ligand de récepteur, qui est sélectif d'endothélium proliférant, de préférence spécifique du complexe VEGF/récepteur de VEGF.

4. Composition pharmaceutique selon la revendication 1, caractérisée en ce que le composant I contient au moins un anticorps monoclonal ou des ligands ou antagonistes contre un antigène provoquant l'apoptose, présent sur des cellules endothéliales proliférantes, de préférence l'intégrine αᵥβ₃.

5. Composition pharmaceutique selon la revendication 1, caractérisée en ce que le composant I contient au moins un immunoconjugué cytotoxique.

6. Composition pharmaceutique selon la revendication 1, caractérisée en ce que le composant I contient au moins une toxine-ligand.

7. Composition pharmaceutique selon la revendication 1, caractérisée en qu'elle contient une substance inhibitrice du métabolisme de cellules tumorales ou de cellules endothéliales.

8. Composition pharmaceutique selon la revendication 1, caractérisée en ce que l'on utilise comme composant II le précurseur de médicament N-(4-β-glucuronyl-3-nitrobenzyloxycarbonyl)doxorubicine.

9. Utilisation d'une ou plusieurs substances actives qui produisent la nécrose dans des tumeurs ou dans des maladies inflammatoires (composant I) avec une ou plusieurs substances actives qui sont activées par les nécroses produites (composant II), pour la fabrication d'un médicament destiné à la lutte contre des tumeurs ou des maladies inflammatoires.
